(19) Europäisches Patentamt
European Patent Office
Office européen des brevets

(11) Veröffentlichungsnummer : **0 001 098**
**B1**

(12) # EUROPÄISCHE PATENTSCHRIFT

(45) Veröffentlichungstag der Patentschrift : **15.04.81**

(51) Int. Cl.³ : **C 07 D417/12**

(21) Anmeldenummer : **78100802.4**

(22) Anmeldetag : **01.09.78**

(54) **Verfahren zur Herstellung von Morpholino-dithio-thiazolen.**

(30) Priorität : **14.09.77 DE 2741261**

(43) Veröffentlichungstag der Anmeldung : **21.03.79 (Patentblatt 79/06)**

(45) Bekanntmachung des Hinweises auf die Patenterteilung : **15.04.81 Patentblatt 81/15**

(84) Benannte Vertragsstaaten : **BE DE FR GB NL**

(56) Entgegenhaltungen :
**DE - A - 2 164 480**
**GB - A - 1 036 345**
**US - A - 3 062 825**

(73) Patentinhaber : **BAYER AG**
**Zentralbereich Patente, Marken und Lizenzen**
**D-5090 Leverkusen 1, Bayerwerk (DE)**

(72) Erfinder : **Steinacker, Karl-Heinz, Dr.**
**Elisabeth-Länggässer-Strasse 9**
**D-5090 Leverkusen (DE)**
Erfinder : **Barnikel, Carl-Dieter, Dr.**
**Rybniker Strasse 10**
**D-5000 Köln 80 (DE)**
Erfinder : **Nierth, Alfred, Dr.**
**Carl-Duisberg-Strasse 329**
**D-5090 Leverkusen 1 (DE)**
Erfinder : **Finzenhagen, Manfred, Dr.**
**Roggendorfstrasse 59**
**D-5000 Köln 80 (DE)**

EP 0 001 098 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

Verfahren zur Herstellung von Morpholinodithiothiazolen

Die Erfindung betrifft ein Verfahren zur Herstellung von Morpholinodithiothiazolen durch Umsetzung einer wässrigen Mercaptothiazolsalzlösung, Morpholin und Schwefel mit einem Oxidationsmittel unter Zugabe von Säure.

Aus der DE-AS 1 138 054 ist es bekannt, Morpholindithiothiazol aus Mercaptothiazolen, Morpholin und Schwefel in Alkohol mit Natriumhypochlorit herzustellen. Bei dieser Reaktion ist ein Überschuss an Amin notwendig, so dass dieses Reagenz nicht voll ausgenutzt wird.

Nach dem Verfahren der DE-OS 2 238 516 wird ein Gemisch von Mercaptobenzthiazol (MBT), Morpholin, Schwefel und Lösungsmittel mit Natriumhypochloritlösung (Bleichlauge) behandelt, wobei jedoch 150 Gew.-Teile Wasser pro 100 Gew.-Teile trockenem MBT nicht überstiegen werden darf.

Aus der US-PS 3 281 418 ist ein ähnliches Verfahren zur Herstellung von Morpholinodithiothiazolen bekannt, bei dem in einem inerten organischen Lösungsmittel 2-Mercaptobenzthiazol oder 2,2'-bis(benzothiazolyl) disulfid, Morpholin und Schwefel zusammen mit der wässrigen Lösung eines wasserlöslichen Oxidationsmittels erhitzt werden. Dabei muss die Reaktionstemperatur oberhalb der Kristallisationstemperatur des Reaktionsgemisches liegen, und das Oxidationsmittel muss vor allem sehr rasch vor der Kristallisation des entstehenden Morpholinodithiothiazols zugegeben werden.

Es wurde nun überraschend gefunden, dass sich Morpholindithiothiazole in überwiegend wässrigem Medium in einem Einstufenprozess aus einem Mercaptothiazolsalz, Morpholin und Schwefel durch direkte Oxidation in guten Ausbeuten herstellen lassen, wenn dem Gemisch in Verlaufe der Reaktion eine Säure zugefügt wird. Für die Reaktion wird die wässrige Lösung eines Salzes eines Mercaptothiazols eingesetzt. Das Reaktionsgemisch enthält einen Katalysator und kann außerdem noch untergeordnete Mengen eines organischen Lösungsmittels enthalten.

Gegenstand der Erfindung ist somit ein Verfahren zur Herstellung von Morpholindithiothiazolen der allgemeinen Formel I

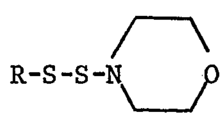 (I)

worin

R einen Benzthiazol-2- oder Naphthiazol-2-Rest bedeutet, der gegebenenfalls durch geradkettige oder verzweigte Alkylgruppen mit 1-10 C-Atomen substituiert sein kann, durch Umsetzung von Verbindungen der allgemeinen Formel II

$$(R—S—)_n\, M \qquad (II)$$

in denen R die oben angegebene Bedeutung hat,

M ein Metallion des Natriums, Kaliums, Lithiums, Magnesiums, Calciums, Bors oder Aluminiums darstellt, und n, eine ganze Zahl von 1 bis 3 bedeutet, mit Morpholin, Schwefel und einem Oxidationsmittel in Gegenwart eines Katalysators in überwiegend wässrigem Medium, dadurch gekennzeichnet, dass eine wässrige Lösung eines Mercaptothiazolsalzes der oben angegebenen allgemeinen Formel II, eingesetzt und im Verlauf der Reaktion eine Säure zugefügt wird.

Geeignete Verbindungen der allgemeinen Formel II

$$(R—S—)_n\, M \qquad (II)$$

sind beispielsweise solche, in denen R einen unsubstituierten oder mit Methyl-, Äthyl-, Propyl-, iso-Propyl-, Hexyl-, Pentyl-, iso-Octyl-, Nonyl- und Docylgruppen mono-, di- oder gemischtsubstituierten Benzthiazyl- oder Naphthiazyl-Rest und

M ein Metallion des Natriums, Kaliums, Lithiums, Magnesiums, Calciums, Bors oder Aluminiums bedeutet.

Bevorzugt werden die Natriumsalze der entsprechenden Mercaptoverbindungen eingesetzt. Besonders bevorzugt ist das Natriumsalz des Mercaptobenzthiazols.

Die Konzentration der wässrigen Thiazolsalzlösung beträgt 5-60 Gew.-%, bevorzugt 35-55 Gew.-%.

Die Reaktion wird in überwiegend wässrigem Medium durchgeführt. Die Menge an Wasser ist dabei nicht kritisch. Sie kann innerhalb eines weiten Bereichs variiert werden und 50 bis 1 000 Gew.-Tle., bevorzugt 80 bis 300 Gew.-Tle., besonders bevorzugt 100 bis 250 Gew.-Tle., bezogen auf 100 Gew.-Tle. trockenes Mercaptothiazol betragen.

Das Verhältnis von Morpholin und Schwefel zu der Thiazolsalzlösung beträgt im Falle des Morpholins 30-200, bevorzugt 50-150 Gew.-Tle., im Falle des Schwefels 10-30, bevorzugt 15-25 Gew.-Tle., bezogen auf 100 Gew.-Tle. trockenes Mercaptobenzthiazol.

Als Säure kann sowohl eine anorganische als auch eine organische Säure eingesetzt werden. Als anorganische Säuren seien im einzelnen beispielsweise genannt: Salzsäure, Schwefelsäure, Salpetersäure, Phosphorsäure, Fluorwasserstoffsäure, Perchlorsäure.

Als organische Säuren seien im einzelnen beispielsweise genannt: Ameisensäure, Essigsäure, Propionsäure, Oxalsäure, Bernsteinsäure, Zitronensäure, Weinsäure, Phthalsäure, Benzoesäure, Phenylessigsäure. Die Konzentration der zugegebenen Säure liegt bei 5-100 Gew.-%.

Die zugegebene Säuremenge beträgt 0,01-2,0 Mol, bevorzugt 0,6-0,99 Mol, bezogen auf 1 Mol des Mercaptothiazolsalzes. Die Gesamtmenge der Säure kann entweder am Anfang vor der Oxidation zugegeben werden oder es wird ein Teil der Säure am Anfang und der Rest im verlauf der Oxidation zugesetzt. In diesem Fall werden am Anfang 0,05-0,99 Mol Säure pro 1 Mol Mercaptothiazolsalz und der Rest im Verlauf der Oxidation zugegeben. Es ist zu vermuten, dass dabei, im Gegensatz zu den bisher bekannten Verfahren, z.T.. auch eine direkte Oxidation des Mercaptothiazolsalzes unter Bildung des Morpholinodithiothiazols erfolgt. Daneben entsteht das Endprodukt aus dem intermediär gebildeten Mercaptothiazol, wie es die weiter unten stehende Reaktionsgleichung erläutert.

Als Oxidationsmittel sind alle bekannten und üblichen Oxidationsmittel geeignet, vorzugsweise solche, die in Form ihrer wässerigen Lösungen eingesetzt werden können. Beispielsweise seien genannt: Halogene, wie Chlor oder Brom, Wasserstoffperoxid, Natriumhypochlorit und Natriumchlorat. Bevorzugt wird Natriumhypochlorit verwendet.

Das Molverhältnis von Oxidationsmittel zu Mercaptothiazolsalz beträgt 3 : 1 bis 1 : 1, bevorzugt 1,9 : 1 bis 1,2 : 1. Geeignete organische Lösungsmittel, die als Co-Lösungsmittel im Reaktionsgemisch eingesetzt werden können, sind aliphatische, cycloaliphatische und araliphatische Alkohole mit 1-15 Kohlenstoffatomen wie Methanol, Äthanol, Propanol-, iso-Propanol, Butanole, Pentanole, Hexanole, Decanole, Benzylalkohol, Cyclohexanol und Cyclopentanol, aliphatische, cycloaliphatische und aromatische Kohlenwasserstoffe mit 5-15 Kohlenstoffatomen, wie Pentan, Hexan, Heptan, Octan, Dodecan, Cyclohexan, Benzol, Toluol und Xylol sowie Äther und Ester.

Wird ohne Co-Lösungsmittel gearbeitet, hat sich eine geringe Menge eines üblichen Emulgators oder Dispergiermittels als vorteilhaft erwiesen.

Als Katalysatoren werden primäre oder sekundäre aliphatische und cycloaliphatische oder sekundäre heterocyclische Amine, Säureamide oder deren Mischungen eingesetzt.

Bevorzugt werden eingesetzt:

Methylamin, Dimethylamin, Cyclohexylamin, Dicyclohexylamin, Piperidin, Äthylamin, Diäthylamin, Propylamin, Butylamin und Dimethylformamid. Der Zusatz des Katalysators erfolgt in einer Dosierung von 0,01 bis 300 Gew.-Tle., bevorzugt von 0,03 bis 12 Gew.-Tle., bezogen auf 100 Gew.-Tle. der Thiazolverbindungen.

Die Reaktion läuft bei Temperaturen zwischen 20-120 °C ab; zweckmässigerweise hält man die Temperatur zwischen 40 °C und 75 °C. Die Reaktion kann isotherm oder bei unterschiedlichen Temperaturen in einem Temperaturbereich von 20-120 °C durchgeführt werden. Die Obergrenze der Reaktionstemperatur wird dabei durch den Siedepunkt der wäßrigen Phase oder des Lösungsmittelgemisches bestimmt.

Der Zeitpunkt der Kristallisation des entstehenden Morpholinodithiothiazols hat keinen Einfluss auf dessen Reinheit und Ausbeute. Die Kristallisation kann während des gesamtem Verlaufs der Oxidation erfolgen, ohne die Eigenschaften des Produktes nachteilig zu beeinflussen. Die nach dem erfindungsgemässen Verfahren hergestellten Morpholinodithiothiazole werden in guter Ausbeute und hoher Reinheit erhalten.

Das folgende Schema erläutert die Reaktion am Beispiel des Natriumsalzes von Mercaptobenzthiazol unter Verwendung von Natriumhypochlorit als Oxidationsmittel.

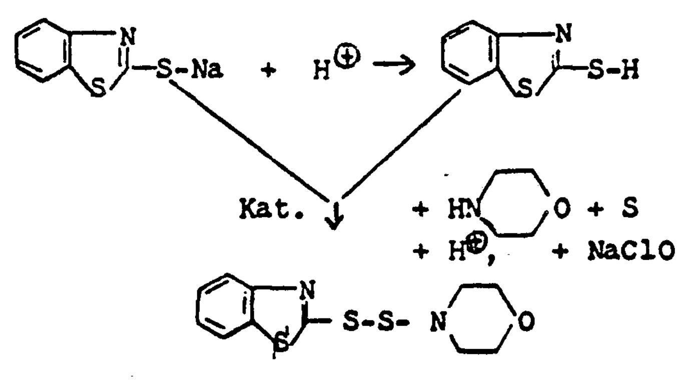


Die erfindungsgemäss hergestellten Verbindungen sind u.a. zur Beschleunigung der Vulkanisation von natürlichen und/oder synthetischen Kautschuken geeignet.

Das bei der Reaktion entstehende Produkt zeichnet sich durch ein feines Korn aus, so daß es sich ohne Mahlung technologisch gut verarbeiten läßt.

In den folgenden Beispielen sind die Prozentangaben stets Gew.-%.

Beispiel 1

In einer Rührapparatur werden vorgelegt :
120,1 g einer 50,2 %igen wässrigen Lösung von Natrium-mercaptobenzthiazol (NaMBT) entsprechend 53,3 g (0,319 Mol) Mercaptobenzthiazol (MBT).

| | |
|---|---|
| 27,7 g | (0,318 Mol) Morpholin |
| 10,0 g | (0,313 Mol) Schwefel |
| 0,65 g | Cyclohexylamin |
| 72 ml | Isopropanol (100 %ig) |
| 13 ml | Wasser |

Bei 59 °C werden 28 ml (0,26 Mol HCl) 30 %ige Salzsäure zugegeben, wonach das Reaktionsgemisch einen pH-Wert von 7,8 hat. Innerhalb von 35 Min. werden dann 215 ml (0,46 Mol) einer etwa 14 Gew.-%igen Natriumhypochloritlösung zugesetzt, wobei nach Zugabe von etwa 125 ml Morpholinodithiobenzthiazol als gelber Niederschlag auszufällen beginnt. Während der Zugabe steigt die Temperatur aus 74 °C.

Nach beendeter Reaktion wird der Niederschlag abfiltriert, 2 Mal mit je 100 ml Isopropanol und 4 Mal mit je 200 ml Wasser gewaschen.

Ausbeute : 75.5 = 83,3 % (bezogen auf eingesetztes MBT)
Schmelzpunkt : 120-131 °C
Reinheit : 95,1 %.

Beispiel 2

In einer Rührapparatur werden vorgelegt :

| | |
|---|---|
| 120,1 g | NaMBT (50,2 gew.-%ige wässrige Lösung) |
| ≙ 53,3 g | MBT (0,319 Mol) |
| 72 ml | Isopropanol 100 %ig |
| 13 ml | Wasser |
| 27,7 g | (0,318 Mol) Morpholin |
| 0,65 g | Cyclohexylamin |
| 10,0 g | (0,313 Mol) Schwefel |

Bei 58 °C werden 15,5 ml (0,147 Mol HCl) 30 %ige Salzsäure zugesetzt (pH = 8,7). Die Lösung enthält auf 100 Gew.-Tle MBT 158 Gew.-Tle Wasser. In Temperaturbereich von 58-73 °C werden in 33 Min. 198 ml (ca. 0,42 Mol) Natriumhypochloritlösung (ca. 14 Gew.-%ig) zudosiert, während parallel 10,2 ml (0,096 Mol HCl) 30 %ige Salzsäure zugesetzt werden. Dabei steigt die Temperatur auf 74 °C.

Nach dem Abkühlen auf Raumtemperatur wird Morpholinodithiobenzthiazol, das nach Zugabe von etwa der Hälfte fer Natriumhypochloritlösung auszufällen begann, abfiltriert, zweimal mit je 100 ml Isopropanol und fünfmal mit je 200 ml Wasser gewaschen und getrocknet.

Ausbeute : 73,6 = 81 % (bezogen auf eingesetztes MBT)
Schmelzpunkt : 129-31 °C
Reinheit : 96,6 %.

Beispiel 3

In einer Rührapparatur wurden vorgelegt :

| | |
|---|---|
| 116,9 g | NaMBT (51,6 gew.-%ige Lösung) |
| ≙ 53,3 g | MBT (0,319 Mol) |
| 55,7 g | Morpholin |
| 0,65 g | Cyclohexylamin |
| 10,0 g | Schwefel |

Bei 58 °C werden 15 g 96 %ige Schwefelsäure (0,29 Mol) zugesetzt. Im Temperaturbereich von 58-74 °C werden 170 ml (0,32 Mol) Natriumhypochloritlösung (ca. 14 Gew.-%ig) zudosiert, wobei nach 140 ml Dosierung Morpholinodithiobenzthiazol als gelber Niederschlag ausfällt. Nach dem Abkühlen wird der Niederschlag abfiltriert und 12 Mol mit 120 ml Wasser gewaschen.

Ausbeute : 84,5-93,3 % (bezogen auf eingesetztes MBT)
Schmelzpunkt : 125-31 °C
Gehalt : 96,1 %.

Beispiele 4 bis 24

Die Beispiele 3 bis 22 zeigen den Effekt der verschiedenen Mengen und Arten der Dosierung von mehreren Säuren auf Ausbeute und Reinheit des Morpholinodithiobenzthiazols. Dabei wird stets von einer wäßrigen Natrium-mercaptobenzthiazollösung ausgegangen (i.a. ca. 50 %ig mit 53,3 g MBT = 0,319 Mol, entsprechend ca. 120 g Lösung). I.a. 85 ml Isopropanol/$H_2O$-Gemisch (entsprechend 85 %igem Azeotrop-Gemisch Isopropanol/$H_2O$), 27,7 g (0,318 Mol) Morpholin, 0,65 g Cyclohexylamin und 10 g Schwefel (0,313 Mol) wurden nacheinander zugesetzt. Nach dem Erwärmen auf 55-60 °C werden entweder die Gesamtmenge oder der erste Teil (s. Tabelle) an Säure zugesetzt. Anschließend wird die Natriumhypochloritlösung (i.a. ein Überschuß von 30-70 Mol-%) in ca. 30 Min. zudosiert, wobei parallel gegebenenfalls der zweite Teil der Säure zugegeben wird. Das Morpholinodithiothiazol fällt i.a. nach der Hälfte der Dosierung als schwachgelb gefärbter Feststoff aus. Die Reaktionsdaten der Beispiele 4 bis 24 sind in der folgenden Tabelle zusammengestellt. Die Tabelle zu sehen p. 6.

## Ansprüche

1. Verfahren zur Herstellung von Morpholinodithiothiazolen der allgemeinen Formel I

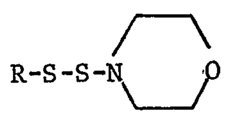

(I)

worin

R einen Benzthiazol-2- oder Naphthiazol-2-Rest bedeutet, der gegebenenfalls durch geradkettige oder verzweigte Alkylgruppen mit 1-10 C-Atomen substituiert sein kann,
durch Umsetzung von Verbindungen der allgemeinen Formel II

$$(R—S—)_n\ M \qquad (II)$$

in denen R die oben angegebene Bedeutung hat,

M ein Metallion des Natriums, Kaliums, Lithiums, Magnesiums, Calciums, Bors oder Aluminiums darstellt, und

n eine ganze Zahl von 1 bis 3 bedeutet,
mit Morpholin, Schwefel und einem Oxidationsmittel in Gegenwart eines Katalysators in überwiegend wäßrigem Medium, dadurch gekennzeichnet, dass eine wässrige Lösung eines Mercaptothiazolsalzes der oben angegebenen allgemeinen Formel II eingesetzt und dem Gemisch im Verlaufe der Reaktion Säure zugefügt wird.

2. Verfahren nach Anspruch 1, dadurch gekennzeichnet, dass die Säuremenge 0,01 bis 2,0 Mol, bezogen auf 1 Mol Mercaptothiazolsalz, beträgt.

3. Verfahren nach Anspruch 1, dadurch gekennzeichnet, dass eine anorganische Säure zugefügt wird.

4. Verfahren nach Anspruch 1, dadurch gekennzeichnet, dass Salzsäure oder Schwefelsäure zugefügt wird.

5. Verfahren nach Anspruch 1, dadurch gekennzeichnet, daß die Gesamtmenge an Säure vor der Oxidation zugeführt wird.

6. Verfahren nach Anspruch 1, dadurch gekennzeichnet, daß vor der Oxidation 0,05 bis 0,99 Mol Säure, bezogen auf 1 Mol Mercaptothiazolsalz, und der Rest der Säure im Verlauf der Oxidation zugefügt wird.

7. Verfahren nach Anspruch 1, dadurch gekennzeichnet, daß ein Natrium- oder Calciumsalz des Mercaptothiazols eingesetzt wird.

8. Verfahren nach Anspruch 1, dadurch gekennzeichnet, daß als Katalysator ein Amin eingesetzt wird.

9. Verfahren nach Anspruch 1, dadurch gekennzeichnet, daß die Reaktion unter Verwendung eines organischen Lösungsmittels als Co-Lösungsmittel erfolgt.

10. Verfahren nach Anspruch 1, dadurch gekennzeichnet, daß als organisches Lösungsmittel ein aliphatischer Alkohol mit 3-5 Kohlenstoffatomen verwendet wird.

11. Verfahren nach Anspruch 1, dadurch gekennzeichnet, daß in Abwesenheit des organischen Lösungsmittels eine kleine Menge eines üblichen Emulgators oder Dispergiermittels eingesetzt wird.

12. Verfahren nach Anspruch 1, dadurch gekennzeichnet, daß die Gewichtsmenge an Morpholin, bezogen auf 100 Gew.-Tle. trockenes Mercaptothiazol, 30-200, bevorzugt 50-150 Gew.-Tle. beträgt.

13. Verfahren nach Anspruch 1, dadurch gekennzeichnet, daß die Gewichtsmenge an Wasser, bezogen auf 100 Gew.-Tle. trockenes Mercaptothiazol, 50 bis 1 000 Gew.-Tle. beträgt.

TABELLE

| Beispiel | 1. Teil Salzsäure (ml) | 2. Teil Salzsäure | Gesamtmenge Säure (Mol/Mol MBT) | Menge $H_2O$ (Gew.Tle/ 100 Gew.Tle. MBT | Schmelzpunkt °C | Reinheit (%) | Ausbeute (%) |
|---|---|---|---|---|---|---|---|
| 4 (1) | 10 | — | 0,29 | 169 | 127-134 | 98,7 | 53,6 |
| 5 (2) | 11,5 | — | 0,34 | 130 | 130-133 | 99,1 | 58,4 |
| 6 | 12,5 | 15 | 0,81 | 153 | 120-125 | 97,9 | 86,7 |
| 7 (2) | 12,5 | 15 | 0,81 | 132 | 129-131 | 96,3 | 84,3 |
| 8 | 15 | 15 | 0,89 | 159 | 119-121 | 92,3 | 83,9 |
| 9 | 15 | 15 | 0,89 | 136 | 129-130 | 95,7 | 83,4 |
| 10 | 22 | — | 0,62 | 170 | 128-132 | 98,8 | 72,4 |
| 11 | 25 | — | 0,74 | 175 | 128-131 | 98,5 | 78,4 |
| 12 | 27 | — | 0,80 | 178 | 128,0 | 97,7 | 81,5 |
| 13 | 23 | 5 | 0,82 | 172 | 125-127 | 92,6 | 82,8 |
| 14 | 29 | — | 0,86 | 181 | 125-132 | 95,7 | 81,7 |
| 15 | 30 | — | 0,89 | 182 | 126-138 | 95,5 | 75,5 |
| 16 | 31 | — | 0,92 | 184 | 122-135 | 90,9 | 84,9 |
| 17 (2) | 33,8 | — | 1,0 | 164 | 120-128 | 81,5 | 84,4 |
| 18 | 22 | 6 | 0,83 | 170 | 129-131 | 98,6 | 81,9 |
| 19 | 24 | 5 | 0,86 | 173 | 128-130 | 97,4 | 75,1 |
| 20 (3) | 23 | — | 0,68 | 175 | 122-128 | 80,6 | 71,7 |
| 21 | 50 g 25,2 %ige $H_2SO_4$ | — | 0,81 | 207 | 130-132 | 98,2 | 79,0 |
| 22 (4) | 13,1 g 96 %ige $H_2SO_4$ | — | 0,80 | 231 | 124-127 | 98,3 | 73,6 |
| 23 (5) | 27 | — | 0,80 | 178 | 123-127 | 97,3 | 84,5 |
| 24 (6) | 98 g 96 %ige $HSO_4$ | — | 0,60 | 113 | 121-130 | 96,4 | 85,3 |

(1) + 10 ml $H_2O$ vor Oxidation.
(2) mit 70 ml Isopropanol statt 85 ml Isopropanol/$H_2O$-Gemisch.
(3) als $Ca(MBT)_2$(22 gew.-%ig an MBT) + 72 Mol Isopropanol 100 %ig.
(4) + 50 ml $H_2O$ vor Oxidation.
(5) + 50 ml Wasser von 60 °C nach Oxidation.
(6) ohne Isopropanol, 41,6 g Morpholin.

## Claims

1. A process for the production of morpholino-dithiothiazoles of the general formula (I) :

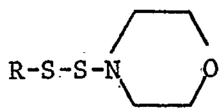 (I)

in which

R represents a 2-benzthiazole or 2-naphthiazole radical which may optionally be substituted by straight-chain or branched-chain alkyl groups containing from 1 to 10 carbon atoms,
by reacting compounds of the general formula (II)

$$(R-S-)_n M \qquad (II)$$

in which

R has the meaning indicated above,

M represents a metal ion of sodium, potassium, lithium, magnesium, calcium, boron or aluminium and

n denotes an integer from 1 to 3,

with morpholine, sulphur and an oxidising agent in the presence of a catalyst in a predominantly aqueous medium, characterised in that an aqueous solution of a mercaptothiazole salt of the above general formula (II) is used and acid is added to the mixture during the course of the reaction.

2. A process according to claim 1, characterised in that the quantity of acid is 0.01 to 2.0 moles based on 1 mole of mercaptothiazole salt.

3. A process according to claim 1, characterised in that an inorganic acid is added.

4. A process according to claim 1, characterised in that hydrochloric acid or sulphuric acid is added.

5. A process according to claim 1, characterised in that the total amount of acid is added before oxidation.

6. A process according to claim 1, characterised in that from 0.05 to 0.99 mole of acid, based on 1 mole of mercaptothiazole salt, is added before oxidation and the remainder of the acid is added during the course of the oxidation.

7. A process according to claim 1, characterized in that a sodium or calcium salt of mercaptothiazole is used.

8. A process according to claim 1, characterised in that an amine is used as catalyst.

9. A process according to claim 1, characterised in that the reaction is carried out using an organic solvent as co-solvent.

10. A process according to claim 1, characterised in that the organic solvent used is an aliphatic alcohol containing from 3 to 5 carbon atoms.

11. A process according to claim 1, characterised in that in the absence of the organic solvent a small amount of a standard emulsifier or dispersant is used.

12. A process according to claim 1, characterised in that the quantity by weight of morpholine, based on 100 parts by weight of dry mercaptothiazole, is 30 to 200, preferably 50 to 150 parts by weight.

13. A process according to claim 1, characterised in that the quantity by weight of water, based on 100 parts by weight of dry mercaptothiazole, is 50 to 1 000 parts by weight.

## Revendications

1. Procédé pour la préparation de morpholinodithiothiazoles de formule générale

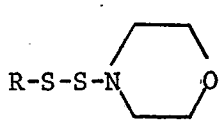 (I)

dans laquelle R représente un reste benzothiazole-2 ou naphtiazole-2, qui peut éventuellement être substitué par des groupes alkyle à chaîne droite ou ramifiée en $C_1$-$C_{10}$,
par réaction de composés de formule générale

$$(R-S-)_n M \qquad (II)$$

dans laquelle R a la signification indiquée ci-dessus,

M représente un ion métallique de sodium, de potassium, de lithium, de magnésium, de calcium, de bore ou d'aluminium et

n représente un nombre entier de 1 à 3,

avec la morpholine, le soufre et un agent oxydant en présence d'un catalyseur dans un milieu essentiellement aqueux, caractérisé en ce que l'on utilise une solution aqueuse d'un sel de mercaptothiazole de formule générale (II) indiquée ci-dessus et on ajoute un acide au cours de la réaction.

2. Procédé selon la revendication 1, caractérisé en ce que la quantité d'acide est de 0,01 à 2,0 moles pour 1 mole de sel de mercaptothiazole.

3. Procédé selon la revendication 1, caractérisé en ce que l'on ajoute un acide inorganique.

4. Procédé selon la revendication 1, caractérisé en ce que l'on ajoute de l'acide chlorhydrique ou de l'acide sulfurique.

5. Procédé selon la revendication 1, caractérisé en ce que la quantité totale d'acide est ajoutée avant l'oxydation.

6. Procédé selon la revendication 1, caractérisé en ce que l'on ajoute 0,05 à 0,99 mole d'acide, pour 1 mole de sel de mercaptothiazole, avant l'oxydation et le reste de l'acide au cours de l'oxydation.

7. Procédé selon la revendication 1, caractérisé en ce que l'on utilise un sel de sodium ou de calcium du mercaptothiazole.

8. Procédé selon la revendication 1, caractérisé en ce que l'on utilise une amine comme catalyseur.

9. Procédé selon la revendication 1, caractérisé en ce que la réaction s'effectue avec utilisation d'un solvant organique comme cosolvant.

10. Procédé selon la revendication 1, caractérisé en ce que l'on utilise comme solvant organique un alcool aliphatique en $C_3$-$C_5$.

11. Procédé selon la revendication 1, caractérisé en ce que l'on utilise une faible quantité d'un émulsifiant ou dispersant habituel en l'absence du solvant organique.

12. Procédé selon la revendication 1, caractérisé en ce que le poids de morpholine est de 30 à 200, de préférence 50 à 150 parties en poids pour 100 parties en poids de mercaptothiazole sec.

13. Procédé selon la revendication 1, caractérisé en ce que la quantité d'eau est de 5 à 1 000 parties en poids pour 100 parties en poids de mercaptothiazole sec.